# EUROPEAN PATENT APPLICATION

(11) **EP 4 049 661 A1**
(43) Date of publication of application: **31.08.2022**
(21) Application number: 20878936.2
(22) Date of filing: 21.10.2020
(51) Int. Cl.: A61K 31/519, A61K 31/53, A61K 45/06, A61P 25/00, C07D 487/04

(54) **USE OF IMIDAZOPYRIMIDINE OR IMIDAZOTRIAZINE COMPOUND FOR PREVENTION, ALLEVIATION, OR TREATMENT OF DEVELOPMENTAL DISABILITY**

(30) Priority: 21.10.2019 KR 20190130397
(71) Applicant: SK Biopharmaceuticals Co., Ltd., Seongnam-si, Gyeonggi-do 13494 (KR)
(72) Inventor: LEE, Ki Ho, Seongnam-si Gyeonggi-do 13494 (KR); CHO, Nahm Ryune, Seongnam-si Gyeonggi-do 13494 (KR); JOUNG, Chan Mi, Seongnam-si Gyeonggi-do 13494 (KR)
(74) Representative: Vossius & Partner Patentanwälte Rechtsanwälte mbB
(86) International application number: PCT/KR2020/014400
(87) International publication number: WO 2021/080313

(57) **Abstract**

The present invention relates to a use, for the prevention, alleviation or treatment of developmental disability, of an imidazopyrimidine or imidazotriazine compound of chemical formula 1, or a pharmaceutically acceptable salt, solvate or hydrate thereof.

## Description

### [Technical Field]

The present invention relates to a use of an imidazopyrimidine or imidazotriazine compound of Chemical Formula 1, or a pharmaceutically acceptable salt, solvate or hydrate thereof for prevention, alleviation or treatment of developmental disorder: 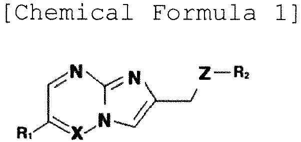 in Chemical Formula 1, X, Z, R₁ and R₂ are as defined herein.

### [Background Art]

Developmental disorder includes, for example, fragile X syndrome (FXS), Angelman syndrome, and Rett syndrome, and treatment for these developmental disorders is limited.

Fragile X syndrome is a genetic disorder, is the most common single genetic cause of autism, and is the genetic cause of intellectual disability, especially among boys. Fragile X syndrome is caused by mutations in the Fmrl (fragile X mental retardation 1) gene found on the X chromosome. The Fmrl gene is the first identified autism-associated gene and encodes fragile X mental retardation protein (FMRP), an RNA-binding protein that regulates translation. In other words, a protein called FMRP is produced by the Fmrl gene, and this protein is necessary for normal brain development, and a lack of this protein causes fragile X syndrome (Source: Center for Disease Control and Prevention). This loss of function typically occurs when there is an expansion of the CGG trinucleotide repeat in the 5' untranslated region of the Fmrl gene. This expansion appears as weak or "fragile-like" ends on the X chromosome.

Fragile X syndrome occurs in both men and women, but the symptoms thereof in women compared to those in men are relatively favorable, and the incidence is also higher in men. According to another report, this disease occurs in 1 in 4,600 men and 1 in 8,000 women (Source: Genetics Home Reference, National Library of Medicine).

Symptoms of fragile X syndrome include developmental delays, learning disability, intellectual disability, socio-behavioral disorder (inability to make eye contact properly, anxiety, problems with attention and concentration, hand flapping, speaking or acting without thinking, hyperlocomotor activity, easily excitable personality), seizures, and the like. In the severity of fragile X syndrome, men have severe intellectual disability, and some women have normal range intelligence or some women have intellectual disability. Autism spectrum disorder frequently occurs in people with fragile X syndrome (Source: Center for Disease Control and Prevention). In addition, there is a risk of seizures in fragile X syndrome, and it is known that about 14% of men and about 4% of women experience seizures (Berry-Kravis et al., 2010, "Seizures in Fragile X Syndrome: Characteristics and Comorbid Diagnoses". Am J Intellect Dev Disabil. 115 (6): 461-72). For the diagnosis of fragile X syndrome, abnormalities in the Fmrl gene are diagnosed through DNA testing using blood.

Angelman syndrome is a disease that occurs when a specific gene on chromosome 15 is not genetically inherited. Symptoms of Angelman syndrome include developmental delays, learning disability, intellectual disability, socio-behavioral disorder (inability to make eye contact properly, anxiety, problems with attention and concentration, hand flapping, speaking or acting without thinking, hyperlocomotor activity, easily excitable personality), seizures, and the like. In addition, autism spectrum disorder frequently occurs in people with Angelman syndrome.

Rett syndrome is a disease caused by genetic mutations in the MECP2 gene. Symptoms of Rett syndrome include developmental delays, learning disability, intellectual disability, socio-behavioral disorder (inability to make eye contact properly, anxiety, problems with attention and concentration, hand flapping, speaking or acting without thinking, hyperlocomotor activity, easily excitable personality), seizures, and the like. In addition, people with Rett syndrome have symptoms similar to those of autism spectrum disorder.

In addition, representative developmental disorders such as pervasive developmental disorder, autism, autistic spectrum disorder, fragile X-associated tremor/ataxia syndrome (FXTAS), Asperger's syndrome, childhood disintegrative disorder, Landau-Kleffner syndrome, Prader-Willi syndrome, 22q11.2 deletion syndrome, tardive dyskinesia, seizure disorder, and Williams syndrome also exhibit symptoms common with fragile X syndrome, Angelman syndrome, and Rett syndrome. In other words, developmental disorders including fragile X syndrome, Angelman syndrome, and Rett syndrome commonly exhibit symptoms such as developmental delays, learning disability, intellectual disability, socio-behavioral disorder (inability to make eye contact properly, anxiety, problems with attention and concentration, hand flapping, speaking or acting without thinking, hyperlocomotor activity, easily excitable personality), and seizures.

Suitable therapeutic agents for such developmental disorders have not been developed to date.

### [Disclosure of Invention]

### [Technical Problem]

An object of the present invention is to provide a method for prevention, alleviation or treatment of developmental disorder.

Another object of the present invention is to provide a use of an imidazopyrimidine or imidazotriazine compound of Chemical Formula 1, or a pharmaceutically acceptable salt, solvate or hydrate thereof for prevention, alleviation or treatment of developmental disorder. 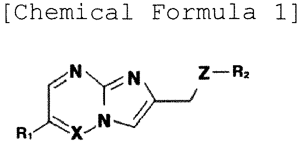 In Chemical Formula 1, X, Z, R₁ and R₂ are as defined herein.

### [Solution to Problem]

The present invention provides a medicament for prevention, alleviation or treatment of developmental disorder, which comprises a therapeutically effective amount of an imidazopyrimidine or imidazotriazine compound of Chemical Formula 1, or a pharmaceutically acceptable salt, solvate or hydrate thereof: 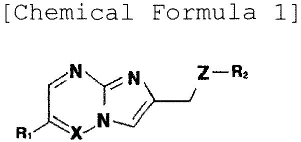 in Chemical Formula 1,
X is CH or N;
Z is 0 or S;
R₁ is C₆-C₁₂ aryl unsubstituted or substituted with one or more substituents selected from halo, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-Ci-Cs alkyl, C₁-C₅ alkoxy-C₁-C₅ alkyl, carbamoyloxy-C₁-C₅ alkyl, C₁-C₅ alkyl-C(O)O-C₁-C₅ alkyl, a 5- or 6-membered heterocycloalkyl-Ci-C₅ alkyl having 1 to 3 heteroatoms selected from N, O and S, and di(C₁-C₅ alkyl) amino-C₁-C₅ alkyl; or 5- to 12-membered unsaturated heterocyclyl having 1 to 5 heteroatoms selected from N, 0 and S, unsubstituted or substituted with one or more substituents selected from halo, hydroxy, C₁-C₅ alkyl, C₁-C₅ alkoxy, and halo-C₁-C₅ alkyl;
R₂ is C₆-C₁₂ aryl unsubstituted or substituted with one or more substituents selected from halo, deuterium, hydroxy and C₁-C₅ alkyl; or 5- to 12-membered unsaturated heterocyclyl having 1 to 3 heteroatoms selected from N, O and S, unsubstituted or substituted with one or more substituents selected from halo and C₁-C₅ alkyl.

The present invention also provides a pharmaceutical composition for alleviation or treatment of fragile X syndrome, which comprises a therapeutically effective amount of the imidazopyrimidine or imidazotriazine compound of Chemical Formula 1, or a pharmaceutically acceptable salt, solvate or hydrate thereof, and additionally one or more pharmaceutically acceptable carriers.

The present invention also provides a method for alleviation or treatment of fragile X syndrome, which comprises administering to a subject a therapeutically effective amount of the imidazopyrimidine or imidazotriazine compound of Chemical Formula 1, or a pharmaceutically acceptable salt, solvate or hydrate thereof.

The present invention also provides use of the imidazopyrimidine or imidazotriazine compound of Chemical Formula 1, or a pharmaceutically acceptable salt, solvate or hydrate thereof for alleviation or treatment of fragile X syndrome.

According to an embodiment of the present invention, R₁ in Chemical Formula 1 is phenyl unsubstituted or substituted with 1 to 3 substituents selected from halo, hydroxy, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-Ci-Cs alkyl, carbamoyloxy-C₁-C₅ alkyl and C₁-C₅ alkyl-C(O)O-C₁-C₅ alkyl; or 5- to 10-membered unsaturated heterocyclyl having 1 to 3 heteroatoms selected from N, O and S, unsubstituted or substituted with 1 to 3 substituents selected from halo, hydroxy, C₁-C₅ alkyl, C₁-C₅ alkoxy and halo-C₁-C₅ alkyl.

According to another embodiment of the present invention, R₂ in Chemical Formula 1 is phenyl unsubstituted or substituted with 1 to 5 substituents selected from halo, deuterium, hydroxy and C₁-C₅ alkyl; or 5- or 6-membered heteroaryl having 1 to 3 heteroatoms selected from N, O and S, unsubstituted or substituted with 1 to 3 substituents selected from halo and C₁-C₅ alkyl.

According to another embodiment of the present invention, in Chemical Formula 1,
X is CH or N;
Z is O;
R₁ is phenyl unsubstituted or substituted with 1 to 3 substituents selected from halo, hydroxy, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-Ci-Cs alkyl, C₁-C₅ alkoxy-C₁-C₅ alkyl, carbamoyloxy-C₁-C₅ alkyl and C₁-C₅ alkyl-C(O)O-C₁-C₅ alkyl; or 5- to 9-membered unsaturated heterocyclyl having 1 or 2 heteroatoms selected from N, O and S, unsubstituted or substituted with 1 or 2 substituents selected from halo, hydroxy, C₁-C₅ alkyl, C₁-C₅ alkoxy and halo-C₁-C₅ alkyl; and
R₂ is phenyl unsubstituted or substituted with 1 to 5 substituents selected from halo, deuterium, hydroxy and C₁-Cs alkyl; or 6-membered heteroaryl having 1 or 2 nitrogen atoms, unsubstituted or substituted with 1 or 2 substituents selected from halo and C₁-C₅ alkyl.

According to another embodiment of the present invention, in Chemical Formula 1,
R₁ is phenyl unsubstituted or substituted with 1 to 3 substituents selected from halo, hydroxy, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl and C₁-C₅ alkyl-C(O)O-C₁-C₅ alkyl; 1,3-benzodioxolyl unsubstituted or substituted with 1 or 2 halo; or pyridyl or pyrimidinyl unsubstituted or substituted with 1 or 2 substituents selected from halo, hydroxy, C₁-C₅ alkyl, C₁-C₅ alkoxy and halo-C₁-C₅ alkyl, and
R₂ is phenyl unsubstituted or substituted with 1 to 5 substituents selected from halo, deuterium, hydroxy and C₁-C₅ alkyl; or pyridyl unsubstituted or substituted with 1 or 2 substituents selected from halo and C₁-C₅ alkyl.

According to another embodiment of the present invention, in Chemical Formula 1,
X is CH;
Z is 0;
R₁ is phenyl unsubstituted or substituted with 1 to 3 substituents selected from halo, hydroxy, C₁-C₅ alkyl, C₁-C₅ alkoxy, C₁-C₅ alkylthio, amino, halo-C₁-C₅ alkyl, hydroxy-C₁-C₅ alkyl and C₁-C₅ alkoxy-C₁-C₅ alkyl; and
R₂ is pyridyl unsubstituted or substituted with 1 or 2 substituents selected from halo and C₁-C₅ alkyl.

Representatives of the compound of Chemical Formula 1 according to the present invention include:
6-(2-fluorophenyl)-2-phenoxymethylimidazo[1,2-a]pyrimidine;
2-phenoxymethyl-6-phenylimidazo[1,2-a]pyrimidine; 6-(2,4-difluorophenyl)-2-phenoxymethylimidazo[1,2-a]pyrimidine;
6-(2-methoxyphenyl)-2-phenoxymethylimidazo[1,2-a]pyrimidine;
6-(2-methylphenyl)-2-phenoxymethylimidazo[1,2-a]pyrimidine;
6-(4-fluoro-2-methylphenyl)-2-phenoxymethylimidazo[1,2-a]pyrimidine;
6-(2,3-difluorophenyl)-2-phenoxymethylimidazo[1,2-a]pyrimidine;
6-(4-fluorophenyl)-2-phenoxymethylimidazo[1,2-a]pyrimidine;
6-(3-aminophenyl)-2-phenoxymethylimidazo[1,2-a]pyrimidine;
6-(2-aminophenyl)-2-phenoxymethylimidazo[1,2-a]pyrimidine;
6-(3-amino-6-methylphenyl)-2-phenoxymethylimidazo[1,2-a]pyrimidine;
6-(3-amino-4-fluorophenyl)-2-phenoxymethylimidazo[1,2-a]pyrimidine;
6-(3-chloro-4-fluorophenyl)-2-phenoxymethylimidazo[1,2-a]pyrimidine;
6-(2-dimethylaminophenyl)-2-phenoxymethylimidazo[1,2-a]pyrimidine;
6-(2-chloro-4-fluorophenyl)-2-phenoxymethylimidazo[1,2-a]pyrimidine;
6-(2-hydroxyphenyl)-2-phenoxymethylimidazo[1,2-a]pyrimidine;
6-(3-hydroxyphenyl)-2-phenoxymethylimidazo[1,2-a]pyrimidine;
6-(4-fluoro-2-trifluoromethylphenyl)-2-phenoxymethylimidazo[1,2-a]pyrimidine;
6-(3,4-difluorophenyl)-2-phenoxymethylimidazo[1,2-a]pyrimidine;
6-(2-methoxy-4-fluorophenyl)-2-phenoxymethylimidazo[1,2-a]pyrimidine;
6-(4-fluoro-3-methoxyphenyl)-2-phenoxymethylimidazo[1,2-a]pyrimidine;
6-(4-chloro-2-methoxyphenyl)-2-phenoxymethylimidazo[1,2-a]pyrimidine;
6-(4-cyano-2-methoxyphenyl)-2-phenoxymethylimidazo[1,2-a]pyrimidine;
6-(7-fluorobenzo[1,3]dioxol-4-yl)-2-phenoxymethylimidazo[1,2-a]pyrimidine;
6-(4-fluoro-2-hydroxymethylphenyl)-2-phenoxymethylimidazo[1,2-a]pyrimidine;
6-(4-fluoro-2-methylthiophenyl)-2-phenoxymethylimidazo[1,2-a]pyrimidine;
6-(2-amino-4-fluorophenyl)-2-phenoxymethylimidazo[1,2-a]pyrimidine;
6-(2,4-difluoro-5-methoxyphenyl)-2-phenoxymethylimidazo[1,2-a]pyrimidine;
6-(2-fluoropyridin-3-yl)-2-phenoxymethylimidazo[1,2-a]pyrimidine;
6-(6-methoxypyridin-3-yl)-2-phenoxymethylimidazo[1,2-a]pyrimidine;
6-(6-fluoropyridin-3-yl)-2-phenoxymethylimidazo[1,2-a]pyrimidine;
6-(4-methylpyridin-3-yl)-2-phenoxymethylimidazo[1,2-a]pyrimidine;
6-(2,6-difluoropyridin-3-yl)-2-phenoxymethylimidazo[1,2-a]pyrimidine;
6-(6-chloropyridin-3-yl)-2-phenoxymethylimidazo[1,2-a]pyrimidine;
6-(2-fluoropyridin-4-yl)-2-phenoxymethylimidazo[1,2-a]pyrimidine;
6-(3-chloropyridin-4-yl)-2-phenoxymethylimidazo[1,2-a]pyrimidine;
6-(4-chlorophenyl)-2-phenoxymethylimidazo[1,2-a]pyrimidine;
6-(6-fluoro-4-methyl-3-pyridyl)-2-phenoxymethylimidazo[1,2-a]pyrimidine;
6-(6-fluoro-5-methyl-3-pyridyl)-2-phenoxymethylimidazo[1,2-a]pyrimidine;
6-(5-fluoro-2-pyridyl)-2-phenoxymethylimidazo[1,2-a]pyrimidine;
6-(2,4-difluorophenyl)-2-(3-fluorophenoxymethyl)imidazo[1,2-a]pyrimidine;
6-(4-fluoro-2-methoxyphenyl)-2-(3-fluorophenoxymethyl)imidazo[1,2-a]pyrimidine;
6-(4-fluoro-3-hydroxyphenyl)-2-(3-fluorophenoxymethyl)-imidazo[1,2-a]pyrimidine;
6-(2-aminophenyl)-2-(3-fluorophenoxymethyl)imidazo[1,2-a]pyrimidine;
6-(3-chloropyridin-4-yl)-2-(3-fluorophenoxymethyl)imidazo[1,2-a]pyrimidine;
6-(4-methylpyridin-3-yl)-2-(3-fluorophenoxymethyl)imidazo[1,2-a]pyrimidine;
6-(2,4-difluorophenyl)-2-(4-fluorophenoxymethyl)imidazo[1,2-a]pyrimidine;
6-(4-fluoro-2-methylphenyl)-2-(4-fluorophenoxymethyl)imidazo[1,2-a]pyrimidine;
6-(3-chloropyridin-4-yl)-2-(4-fluorophenoxymethyl)imidazo[1,2-a]pyrimidine;
6-(2,6-dimethylphenyl)-2-(4-fluorophenoxymethyl)imidazo[1,2-a]pyrimidine;
2-[(4-fluorophenoxy)methyl]-6-(6-fluoro-3-pyridyl)imidazo[1,2-a]pyrimidine;
4-[[6-(4-fluorophenyl)imidazo[1,2-a]pyrimidin-2-yl]methoxy]phenol;
2-[(4-fluorophenoxy)methyl]-6-(4-fluorophenyl)imidazo[1,2-a]pyrimidine;
2-[(3-fluorophenoxy)methyl]-6-(4-fluorophenyl)imidazo[1,2-a]pyrimidine;
2-fluoro-5-[2-[(4-fluorophenoxy)methyl]imidazo[1,2-a]pyrimidin-6-yl]phenol;
2-[(4-fluorophenoxy)methyl]-6-phenyl-imidazo[1,2-a]pyrimidine;
5-fluoro-2-[2-[(3-fluorophenoxy)methyl]imidazo[1,2-a]pyrimidin-6-yl]phenol;
5-fluoro-2-[2-[(4-fluorophenoxy)methyl]imidazo[1,2-a]pyrimidin-6-yl]phenol;
6-(4-fluorophenyl)-2-[(2,3,4,5,6-pentadeuteriophenoxy)methyl]imidazo[1,2-a]pyrimidine;
2-[(4-fluorophenoxy)methyl]6-(o-tolyl)imidazo[1,2-a]pyrimidine;
6-(5-fluoro-2-methyl-phenyl)-2-[(4-fluorophenoxy)methyl]imidazo[1,2-a]pyrimidine;
2-[(4-fluorophenoxy)methyl]-6-(2-fluoro-4-pyridyl)imidazo[1,2-a]pyrimidine;
2-[2-[(4-fluorophenoxy)methyl]imidazo[1,2-a]pyrimidin-6-yl]phenol;
6-(2-fluoro-4-methyl-phenyl)-2-[(4-fluorophenoxy)methyl]imidazo[1,2-a]pyrimidine;
2-[(4-fluorophenoxy)methyl]-6-[6-(trifluoromethyl)-3-pyridyl]imidazo[1,2-a]pyrimidine;
2-[2-[(4-fluorophenoxy)methyl]imidazo[1,2-a]pyrimidin-6-yl]aniline;
6-(2-chloro-4-fluoro-phenyl)-2-[(4-fluorophenoxy)methyl]imidazo[1,2-a]pyrimidine;
4-[2-[(4-fluorophenoxy)methyl]imidazo[1,2-a]pyrimidin-6-yl]-3-methoxy-benzonitrile;
6-(4-chloro-2-methoxy-phenyl)-2-[(4-fluorophenoxy)methyl]imidazo[1,2-a]pyrimidine;
2-fluoro-5-[2-[(4-fluorophenoxy)methyl]imidazo[1,2-a]pyrimidin-6-yl]aniline;
6-(2,6-difluoro-3-pyridyl)-2-[(3-fluorophenoxy)methyl]imidazo[1,2-a]pyrimidine;
5-[2-[(4-fluorophenoxy)methyl]imidazo[1,2-a]pyrimidin-6-yl]-2-methyl-aniline;
4,5-difluoro-2-[2-[(4-fluorophenoxy)methyl]imidazo[1,2-a]pyrimidin-6-yl]aniline;
2-[2-[(4-fluorophenoxy)methyl]imidazo[1,2-a]pyrimidin-6-yl]-5-methyl-aniline;
5-chloro-2-[2-[(4-fluorophenoxy)methyl]imidazo[1,2-a]pyrimidin-6-yl]aniline;
2-[2-[(4-fluorophenoxy)methyl]imidazo[1,2-a]pyrimidin-6-yl]-4-methyl-aniline;
5-fluoro-2-[2-[(4-fluorophenoxy)methyl]imidazo[1,2-a]pyrimidin-6-yl]aniline;
2-[(4-fluorophenoxy)methyl]-6-(4-methyl-3-pyridyl)imidazo[1,2-a]pyrimidine;
2-[(3-fluorophenoxy)methyl]-6-(2-fluoro-4-pyridyl)imidazo[1,2-a]pyrimidine;
2-[(3-fluorophenoxy)methyl]-6-[6-(trifluoromethyl)-3-pyridyl)imidazo[1,2-a]pyrimidine;
2-[(3-fluorophenoxy)methyl]-6-(6-fluoro-3-pyridyl)imidazo[1,2-a]pyrimidine;
2-[(3-fluorophenoxy)methyl]-6-(2-fluoro-3-pyridyl)imidazo[1,2-a]pyrimidine;
6-(5,6-difluoro-3-pyridyl)-2-[(4-fluorophenoxy)methyl]imidazo[1,2-a]pyrimidine;
5-[2-[(4-fluorophenoxy)methyl]imidazo[1,2-a]pyrimidin-6-yl]-2-methoxy-aniline;
2-[(4-fluorophenoxy)methyl]-6-(5-fluoro-2-pyridyl)imidazo[1,2-a]pyrimidine;
2-[(4-fluorophenoxy)methyl]-6-(5-methoxy-2-pyridyl)imidazo[1,2-a]pyrimidine;
6-(4-chloro-3-pyridyl)-2-[(4-fluorophenoxy)methyl]imidazo[1,2-a]pyrimidine;
6-(5-chloro-3-pyridyl)-2-[(4-fluorophenoxy)methyl]imidazo[1,2-a]pyrimidine;
2-[(4-fluorophenoxy)methyl]-6-(5-methoxy-3-pyridyl)imidazo[1,2-a]pyrimidine;
5-[2-[(4-fluorophenoxy)methyl]imidazo[1,2-a]pyrimidin-6-yl]pyridin-2-ol;
6-(6-fluoro-5-methyl-3-pyridyl)-2-[(4-fluorophenoxy)methyl]imidazo[1,2-a]pyrimidine;
2-[(4-fluorophenoxy)methyl]-6-(6-methyl-3-pyridyl)imidazo[1,2-a]pyrimidine;
2-[(3-fluorophenoxy)methyl]-6-(5-fluoro-2-pyridyl)imidazo[1,2-a]pyrimidine;
2-[(3-fluorophenoxy)methyl]-6-[4-fluoro-2-(trifluoromethyl)phenyl]imidazo[1,2-a]pyrimidine;
4-[2-[(3-fluorophenoxy)methyl]imidazo[1,2-a]pyrimidin-6-yl]-3-methoxy-benzonitrile;
[5-fluoro-2-[2-[(3-fluorophenoxy)methyl]imidazo[1,2-a]pyrimidin-6-yl]phenyl]methanol;
[5-fluoro-2-[2-[(4-fluorophenoxy)methyl]imidazo[1,2-a]pyrimidin-6-yl]phenyl]methanol;
6-(4-fluoro-2-methylsulfanyl-phenyl)-2-[(4-fluorophenoxy)methyl]imidazo[1,2-a]pyrimidine;
2-[(4-fluorophenoxy)methyl]-6-(2-methoxy-4-pyridyl)imidazo[1,2-a]pyrimidine;
2-[2-[(3-fluorophenoxy)methyl]imidazo[1,2-a]pyrimidin-6-yl]-4-methyl-aniline;
5-fluoro-2-[2-[(3-fluorophenoxy)methyl]imidazo[1,2-a]pyrimidin-6-yl]aniline;
6-(4-fluorophenyl)-2-(pyridin-2-yloxymethyl)imidazo[1,2-a]pyrimidine;
6-(2-methylphenyl)-2-(pyridin-2-yloxymethyl)imidazo[1,2-a]pyrimidine;
6-(4-fluoro-2-methoxyphenyl)-2-(pyridin-2-yloxymethyl)imidazo[1,2-a]pyrimidine;
6-(2,4-difluorophenyl)-2-(pyridin-2-yloxymethyl)imidazo[1,2-a]pyrimidine;
6-(4-fluoro-2-methylphenyl)-2-(pyridin-2-yloxymethyl)imidazo[1,2-a]pyrimidine;
6-(2,3-difluorophenyl)-2-(pyridin-2-yloxymethyl)imidazo[1,2-a]pyrimidine;
6-(5-fluoro-2-methylphenyl)-2-(pyridin-2-yloxymethyl)imidazo[1,2-a]pyrimidine;
6-(3-fluoro-2-methylphenyl)-2-(pyridin-2-yloxymethyl)imidazo[1,2-a]pyrimidine;
6-(7-fluoro-2H-benzo[1,3]dioxol-4-yl)-2-(pyridin-2-yloxymethyl)imidazo[1,2-a]pyrimidine;
6-(4-chloro-2-methoxyphenyl)-2-(pyridin-2-yloxymethyl)imidazo[1,2-a]pyrimidine;
6-(3-fluoro-2-methoxy-phenyl)-2-(2-pyridyloxymethyl)imidazo[1,2-a]pyrimidine;
6-[4-fluoro-2-(trifluoromethyl)phenyl]-2-(2-pyridyloxymethyl)imidazo[1,2-a]pyrimidine;
6-(4-fluoro-2-methyl-phenyl)-2-[(5-fluoro-2-pyridyl)oxymethyl)imidazo[1,2-a]pyrimidine;
6-(2-ethylphenyl)-2-(2-pyridyloxymethyl)imidazo[1,2-a]pyrimidine;
6-(4-fluoro-2-methyl-phenyl)-2-[(4-fluoro-2-pyridyl)oxymethyl)imidazo[1,2-a]pyrimidine;
6-(2-fluoro-4-methyl-phenyl)-2-(2-pyridyloxymethyl)imidazo[1,2-a]pyrimidine;
6-(4-fluoro-2-methoxy-phenyl)-2-[(4-fluoro-2-pyridyl)oxymethyl)imidazo[1,2-a]pyrimidine;
2-[(5-fluoro-2-pyridyl)oxymethyl]-6-[4-fluoro-2-(trifluoromethyl)phenyl]imidazo[1,2-a]pyrimidine;
6-(2,4-difluorophenyl)-2-[(4-fluoro-2-pyridyl)oxymethyl)imidazo[1,2-a]pyrimidine;
6-(3-fluoro-2-methoxy-phenyl)-2-[(4-fluoro-2-pyridyl)oxymethyl)imidazo[1,2-a]pyrimidine;
6-(2,3-difluorophenyl)-2-[(4-fluoro-2-pyridyl)oxymethyl)imidazo[1,2-a]pyrimidine;
6-(4-fluorophenyl)-2-[(4-fluoro-2-pyridyl)oxymethyl)imidazo[1,2-a]pyrimidine;
6-(3-fluoro-2-methyl-phenyl)-2-[(5-fluoro-2-pyridyl)oxymethyl)imidazo[1,2-a]pyrimidine;
2-[(4-fluoro-2-pyridyl)oxymethyl]-6-[4-fluoro-2-(trifluoromethyl)phenyl]imidazo[1,2-a]pyrimidine;
2-[(5-fluoro-2-pyridyl)oxymethyl]-6-(o-tolyl)imidazo[1,2-a]pyrimidine;
6-(4-chloro-2-methyl-phenyl)-2-[(5-fluoro-2-pyridyl)oxymethyl)imidazo[1,2-a]pyrimidine;
6-(2,4-dimethylphenyl)-2-[(5-fluoro-2-pyridyl)oxymethyl)imidazo[1,2-a]pyrimidine;
6-(4-fluorophenyl)-2-[(5-fluoro-2-pyridyl)oxymethyl)imidazo[1,2-a]pyrimidine;
2-(2-pyridyloxymethyl)-6-[6-(trifluoromethyl)-3-pyridyl]imidazo[1,2-a]pyrimidine;
2-[(5-fluoro-2-pyridyl)oxymethyl]-6-[6-(trifluoromethyl)-3-pyridyl]imidazo[1,2-a]pyrimidine;
6-(5-fluoro-2-pyridyl)-2-(2-pyridyloxymethyl)imidazo[1,2-a]pyrimidine;
2-fluoro-5-[2-[(5-fluoro-2-pyridyl)oxymethyl]imidazo[1,2-a]pyrimidin-6-yl]aniline;
2-fluoro-5-[2-(2-pyridyloxymethyl)imidazo[1,2-a]pyrimidin-6-yl]aniline;
[5-fluoro-2-[2-(2-pyridyloxymethyl)imidazo[1,2-a]pyrimidin-6-yl]phenyl]methanol;
3-methoxy-4-[2-(2-pyridyloxymethyl)imidazo[1,2-a]pyrimidin-6-yl]benzonitrile;
4-[2-[(5-fluoro-2-pyridyl)oxymethyl]imidazo[1,2-a]pyrimidin-6-yl]-3-methoxy-benzonitrile;
6-(4-fluoro-2-methylsulfanyl-phenyl)-2-(2-pyridyloxymethyl)imidazo[1,2-a]pyrimidine;
[5-fluoro-2-[2-[(5-fluoro-2-pyridyl)oxymethyl]imidazo[1,2-a]pyrimidin-6-yl]phenyl]methanol;
4-[2-(2-pyridyloxymethyl)imidazo[1,2-a]pyrimidin-6-yl]benzonitrile;
6-[4-fluoro-2-(methoxymethyl)phenyl]-2-(2-pyridyloxymethyl)imidazo[1,2-a]pyrimidine;
[2-[2-(2-pyridyloxymethyl)imidazo[1,2-a]pyrimidin-6-yl]-5-(trifluoromethyl)phenyl]methanol;
6-(2-isopropylphenyl)-2-(2-pyridyloxymethyl)imidazo[1,2-a]pyrimidine;
4-[2-(2-pyridyloxymethyl)imidazo[1,2-a]pyrimidin-6-yl]-3-(trifluoromethyl)benzaldehyde;
6-[4-chloro-2-(trifluoromethyl)phenyl]-2-(2-pyridyloxymethyl)imidazo[1,2-a]pyrimidine;
6-(4-fluorophenyl)-2-(pyridin-3-yloxymethyl)imidazo[1,2-a]pyrimidine;
6-(4-fluoro-2-methoxyphenyl)-2-(pyridin-3-yloxymethyl)imidazo[1,2-a]pyrimidine;
6-(2,4-difluorophenyl)-2-(pyridin-3-yloxymethyl)imidazo[1,2-a]pyrimidine;
6-(4-fluoro-2-methylphenyl)-2-(pyridin-3-yloxymethyl)imidazo[1,2-a]pyrimidine;
6-(4-fluoro-2-hydroxyphenyl)-2-(pyridin-3-yloxymethyl)imidazo[1,2-a]pyrimidine;
6-(4-fluoro-2-methoxyphenyl)-2-(pyridin-4-yloxymethyl)imidazo[1,2-a]pyrimidine;
6-(2,4-difluorophenyl)-2-(pyridin-4-yloxymethyl)imidazo[1,2-a]pyrimidine;
6-(2-methylphenyl)-2-(5-fluoro-pyridin-3-yloxymethyl)imidazo[1,2-a]pyrimidine;
6-(4-fluoro-2-methylphenyl)-2-(5-fluoro-pyridin-3-yloxymethyl)imidazo[1,2-a]pyrimidine;
6-(4-fluoro-2-methoxyphenyl)-2-(5-fluoropyridin-3-yloxymethyl)imidazo[1,2-a]pyrimidine;
6-(4-fluoro-2-hydroxyphenyl)-2-(5-fluoropyridin-3-yloxymethyl)imidazo[1,2-a]pyrimidine;
6-(2,4-difluorophenyl)-2-(6-fluoropyridin-3-yloxymethyl)imidazo[1,2-a]pyrimidine;
6-(4-fluoro-2-methylphenyl)-2-(6-fluoropyridin-3-yloxymethyl)imidazo[1,2-a]pyrimidine;
6-(4-fluoro-2-methylphenyl)-2-(2-fluoropyridin-3-yloxymethyl)imidazo[1,2-a]pyrimidine;
6-(4-fluoro-2-methylphenyl)-2-(5-chloropyridin-3-yloxymethyl)imidazo[1,2-a]pyrimidine;
6-(2,4-difluorophenyl)-2-(2-fluoropyridin-4-yloxymethyl)imidazo[1,2-a]pyrimidine;
6-(4-fluoro-2-methylphenyl)-2-(2-fluoropyridin-4-yloxymethyl)imidazo[1,2-a]pyrimidine;
6-(4-fluoro-2-methoxyphenyl)-2-(2-fluoropyridin-4-yloxymethyl)imidazo[1,2-a]pyrimidine;
6-(4-fluorophenyl)-2-(2-fluoropyridin-4-yloxymethyl)imidazo[1,2-a]pyrimidine;
6-(2,3-difluorophenyl)-2-(2-fluoropyridin-4-yloxymethyl)imidazo[1,2-a]pyrimidine;
6-(2-fluorophenyl)-2-(2-fluoropyridin-4-yloxymethyl)imidazo[1,2-a]pyrimidine;
6-(4-fluoro-2-hydroxyphenyl)-2-(2-fluoropyridin-4-yloxymethyl)imidazo[1,2-a]pyrimidine;
6-(2-methylphenyl)-2-(2-fluoropyridin-4-yloxymethyl)imidazo[1,2-a]pyrimidine;
6-(2-hydroxyphenyl)-2-(2-fluoropyridin-4-yloxymethyl)imidazo[1,2-a]pyrimidine;
6-(4-fluoro-2-hydroxymethylphenyl)-2-(2-fluoropyridin-4-yloxymethyl)imidazo[1,2-a]pyrimidine;
6-(4-fluorophenyl)-2-[(5-fluoro-3-pyridyl)oxymethyl)imidazo[1,2-a]pyrimidine;
2-[(2-chloro-4-pyridyl)oxymethyl]-6-(4-fluorophenyl)imidazo[1,2-a]pyrimidine;
2-[(5-fluoro-3-pyridyl)oxymethyl]-6-[4-fluoro-2-(trifluoromethyl)phenyl]imidazo[1,2-a]pyrimidine;
2-[(2-fluoro-4-pyridyl)oxymethyl]-6-[4-fluoro-2-(trifluoromethyl)phenyl]imidazo[1,2-a]pyrimidine;
5-fluoro-2-[2-[(5-fluoro-3-pyridyl)oxymethyl]imidazo[1,2-a]pyrimidin-6-yl]aniline;
5-fluoro-2-[2-[(2-fluoro-4-pyridyl)oxymethyl]imidazo[1,2-a]pyrimidin-6-yl]aniline;
[5-fluoro-2-[2-[(5-fluoro-3-pyridyl)oxymethyl]imidazo[1,2-a]pyrimidin-6-yl]phenyl]methanol;
2-(2,4-difluorophenyl)-6-(phenoxymethyl)imidazo[1,2-b][1,2,4]triazine;
2-(2,4-difluorophenyl)-6-((pyridin-4-yloxy)methyl)imidazo[1,2-b][1,2,4]triazine;
2-(2,4-difluorophenyl)-6-((pyridin-2-yloxy)methyl)imidazo[1,2-b][1,2,4]triazine;
2-(4-fluorophenyl)-6-((pyridin-4-yloxy)methyl)imidazo[1,2-b] [1,2,4]triazine;
2-(2-methylphenyl)-6-((pyridin-4-yloxy)methyl)imidazo[1,2-b] [1,2,4]triazine;
2-(2,4-difluorophenyl)-6-((2-fluoropyridin-4-yloxy)methyl)imidazo[1,2-b][1,2,4]triazine;
2-(4-fluoro-2-methylphenyl)-6-((2-fluoropyridin-4-yloxy)methyl)imidazo[1,2-b][1,2,4]triazine;
2-(2-methylphenyl)-6-((2-fluoropyridin-4-yloxy)methyl)imidazo[1,2-b][1,2,4]triazine;
2-[4-fluoro-2-(trifluoromethyl)phenyl]-6-(2-pyridyloxymethyl)imidazo[1,2-b] [1,2,4]triazine;
2-(3-fluoro-2-methyl-phenyl)-6-(2-pyridyloxymethyl)imidazo[1,2-b][1,2,4]triazine;
2-(4-fluoro-2-methyl-phenyl)-6-(2-pyridyloxymethyl)imidazo[1,2-b][1,2,4]triazine;
2-[4-chloro-2-(trifluoromethyl)phenyl]-6-(2-pyridyloxymethyl)imidazo[1,2-b][1,2,4]triazine;
2-(4-chloro-2-methyl-phenyl)-6-(2-pyridyloxymethyl)imidazo[1,2-b][1,2,4]triazine;
2-(4-fluoro-2-methyl-phenyl)-6-(2-pyridyloxymethyl)imidazo[1,2-b][1,2,4]triazine;
2-(4-fluoro-2-methyl-phenyl)-6-[(5-fluoro-2-pyridyl)oxymethyl]imidazo[1,2-b][1,2,4]triazine ;
[5-fluoro-2-[2-[(4-fluorophenoxy)methyl]imidazo[1,2-a]pyrimidin-6-yl]phenyl]methyl carbamate;
[5-fluoro-2-[2-(phenoxymethyl)imidazo[1,2-a]pyrimidin-6-yl]phenyl]methyl carbamate;
[5-fluoro-2-[2-(2-pyridyloxymethyl)imidazo[1,2-a]pyrimidin-6-yl]phenyl]methyl carbamate;
[5-fluoro-2-[2-(2-pyridyloxymethyl)imidazo[1,2-a]pyrimidin-6-yl]phenyl]methyl acetate;
6-[2-(chloromethyl)-4-fluoro-phenyl]-2-[(4-fluorophenoxy)methyl)imidazo[1,2-a]pyrimidine;
6-[4-fluoro-2-(fluoromethyl)phenyl]-2-(2-pyridyloxymethyl)imidazo[1,2-a]pyrimidine; and
6-[4-fluoro-2-(fluoromethyl)phenyl]-2-[(4-fluorophenoxy)methyl]imidazo[1,2-a]pyrimidine.

More specifically, representatives of the compound of Chemical Formula 1 according to the present invention include:
6-(4-fluorophenyl)-2-(pyridin-2-yloxymethyl)imidazo[1,2-a]pyrimidine;
6-[4-fluoro-2-(trifluoromethyl)phenyl]-2-(2-pyridyloxymethyl)imidazo[1,2-a]pyrimidine;
6-(4-fluoro-2-methyl-phenyl)-2-[(5-fluoro-2-pyridyl)oxymethyl)imidazo[1,2-a]pyrimidine;
6-(4-fluorophenyl)-2-[(5-fluoro-2-pyridyl)oxymethyl)imidazo[1,2-a]pyrimidine;
[5-fluoro-2-[2-(2-pyridyloxymethyl)imidazo[1,2-a]pyrimidin-6-yl]phenyl]methanol; and
6-[4-fluoro-2-(fluoromethyl)phenyl]-2-(2-pyridyloxymethyl)imidazo[1,2-a]pyrimidine.

The imidazopyrimidine or imidazotriazine compound of Chemical Formula 1 can be prepared using known compounds or compounds that can be easily prepared therefrom by a person of ordinary skill in the art for compound synthesis. In particular, the method for preparing the compound of Chemical Formula 1 is described in detail in International Patent Publication No. WO 2016/137260 A1, which is incorporated herein by reference. The compound of Chemical Formula 1 may be chemically synthesized by the method described in the above literature, but this is merely to present one exemplary method, and is not intended to limit the scope of the invention as the order of unit operations may be selectively changed as necessary.

The imidazopyrimidine or imidazotriazine compound of Chemical Formula 1 may be used for the prevention, alleviation or treatment of developmental disorder.

The imidazopyrimidine or imidazotriazine compound of Chemical Formula 1 may also be applied to the prevention, alleviation or treatment of symptoms of developmental disorder.

In an embodiment, the developmental disorder is selected from group consisting of pervasive developmental disorder, autism, autistic spectrum disorder, fragile X syndrome, a developmental disorder caused by fragile X syndrome, a developmental disorder exhibiting symptoms similar to those of fragile X syndrome, Angelman syndrome, a developmental disorder caused by Angelman syndrome, a developmental disorder exhibiting symptoms similar to those of Angelman syndrome, Rett syndrome, a developmental disorder caused by Rett syndrome, a developmental disorder exhibiting symptoms similar to those of Rett syndrome, fragile X-associated tremor/ataxia syndrome (FXTAS), Asperger's syndrome, a developmental disorder caused by Asperger's syndrome, a developmental disorder exhibiting symptoms similar to those of Asperger's syndrome, childhood disintegrative disorder, a developmental disorder caused by childhood disintegrative disorder, a developmental disorder exhibiting symptoms similar to those of childhood disintegrative disorder, Landau-Kleffner syndrome, a developmental disorder caused by Landau-Kleffner syndrome, a developmental disorder exhibiting symptoms similar to those of Landau-Kleffner syndrome, Prader-Willi syndrome, a developmental disorder caused by Prader-Willi syndrome, a developmental disorder exhibiting symptoms similar to those of Prader-Willi syndrome, 22q11.2 deletion syndrome, a developmental disorder caused by 22q11.2 deletion syndrome, a developmental disorder exhibiting symptoms similar to those of 22q11.2 deletion syndrome, tardive dyskinesia, a developmental disorder caused by tardive dyskinesia, a developmental disorder exhibiting symptoms similar to those of tardive dyskinesia, seizure disorder, a developmental disorder caused by seizure disorder, a developmental disorder exhibiting symptoms similar to those of seizure disorder, Williams syndrome, a developmental disorder caused by Williams syndrome, and a developmental disorder exhibiting symptoms similar to those of Williams syndrome.

In an embodiment, the developmental disorder is selected from group consisting of fragile X syndrome, a developmental disorder caused by fragile X syndrome, a developmental disorder exhibiting symptoms similar to those of fragile X syndrome, Angelman syndrome, a developmental disorder caused by Angelman syndrome, a developmental disorder exhibiting symptoms similar to those of Angelman syndrome, Rett syndrome, a developmental disorder caused by Rett syndrome, and a developmental disorder exhibiting symptoms similar to those of Rett syndrome.

In an embodiment, the developmental disorder is selected from the group consisting of fragile X syndrome, a developmental disorder caused by fragile X syndrome, and a developmental disorder exhibiting symptoms similar to those of fragile X syndrome.

In an embodiment, a developmental disorder exhibiting symptoms similar to those of fragile X syndrome, a developmental disorder exhibiting symptoms similar to those of Angelman syndrome, a developmental disorder exhibiting symptoms similar to those of Rett syndrome, a developmental disorder exhibiting symptoms disability to those of Asperger's syndrome, a developmental disorder exhibiting symptoms similar to those of childhood disintegrative disorder, a developmental disorder exhibiting symptoms similar to those of Landau-Kleffner syndrome, a developmental disorder exhibiting symptoms similar to those of Prader-Willi syndrome, a developmental disorder exhibiting symptoms similar to those of 22q11.2 deletion syndrome, a developmental disorder exhibiting symptoms similar to those of tardive dyskinesia, a developmental disorder exhibiting symptoms similar to those of seizure disorder, and a developmental disorder exhibiting symptoms similar to those of Williams syndrome may refer to a disease exhibiting symptoms including developmental delays, learning disability, intellectual disability, socio-behavioral disorder (inability to make eye contact properly, anxiety, problems with attention and concentration, hand flapping, speaking or acting without thinking, hyperlocomotor activity, easily excitable personality), and seizures.

In an embodiment, the imidazopyrimidine or imidazotriazine compound of Chemical Formula 1 may be used for prevention, alleviation or treatment of fragile X syndrome.

In an embodiment, the imidazopyrimidine or imidazotriazine compound of Chemical Formula 1 may also be applied to the prevention, alleviation or treatment of symptoms of fragile X syndrome.

Consequently, the medicament or pharmaceutical composition according to an embodiment of the present invention may be used to prevent, alleviate or treat the symptoms of fragile X syndrome, and the symptoms include, but are not limited to, developmental delays, learning disability, socio-behavioral disorder, and seizures.

The medicament or pharmaceutical composition according to an embodiment of the present invention may also be used to prevent, alleviate or treat autism spectrum disorder caused by fragile X syndrome or autism spectrum disorder exhibiting symptoms similar to those of fragile X syndrome.

The efficacy of the compound of Chemical Formula 1 on fragile X syndrome may be verified using a known model. For example, an Fmrl gene knockout mouse model exhibits several clinical symptoms observed in fragile X syndrome and is thus used as a means to verify the drug efficacy for the study of the mechanism of the disease and the development of a therapeutic agent (Bakker et al., 1994, "Fmrl knockout mice: A model to study fragile X mental retardation", Cell, 15;78(1) :23-33) . Typical phenotypes of this mouse model include audiogenic seizure, hyperlocomotor activity, cognitive deficits including visual memory, spatial memory, and fear memory, concentration deficit disorder, and the like (Reinhard et al., Neurobiol Learn Mem. 2019 Oct;164:107042).

The dosage of the imidazopyrimidine or imidazotriazine compound of Chemical Formula 1 for the alleviation or treatment of the disease usually vary depending on the severity of disease, and the body weight and metabolic status of the subject to be treated. A "therapeutically effective amount" for an individual patient means an amount sufficient to achieve the aforementioned pharmacological effect, namely, a therapeutic effect. As the therapeutically effective amount of the compound of Chemical Formula 1, the compound may be contained in the pharmaceutical composition at 0.1 to 500 mg/kg (body weight), preferably 0.5 to 100 mg/kg (body weight) per day when administered to mammals including humans. Such a pharmaceutical composition may be administered one time a day or administered by being divided into two or more times a day.

The compound of the present invention may be administered by conventional methods used for administration of therapeutic agents, such as oral, parenteral, intravenous, intramuscular, subcutaneous or rectal administration.

The medicament or pharmaceutical composition according to an embodiment of the present invention may contain a therapeutically effective amount of a compound selected from the group consisting of the imidazopyrimidine or imidazotriazine compounds of the present invention, pharmaceutically acceptable salts, solvates, and hydrates thereof, and combinations of these.

The pharmaceutically acceptable salt includes both acid or base additional salts and stereochemically isomeric forms thereof. The salt includes any salt that maintains the activity of the parent compound in the object to be administered and does not cause an undesirable effect, and is not particularly limited. Such a salt includes inorganic and organic salts, and may be, for example, salts of acetic acid, nitric acid, aspartic acid, sulfonic acid, sulfuric acid, maleic acid, glutamic acid, formic acid, succinic acid, phosphoric acid, phthalic acid, tannic acid, tartaric acid, hydrobromic acid, propionic acid, benzenesulfonic acid, benzoic acid, stearic acid, esylic acid, lactic acid, bicarbonic acid, bisulfuric acid, bitartaric acid, oxalic acid, butyric acid, calcium edetate, camsylic acid, carbonic acid, chlorobenzoic acid, citric acid, edetic acid, toluenesulfonic acid, edisylic acid, esylic acid, fumaric acid, gluceptic acid, pamoic acid, gluconic acid, glycollylarsanilic acid, methyl nitric acid, polygalactouronic acid, hexylresorcinoic acid, malonic acid, hydrobamic acid, hydrochloric acid, hydroiodic acid, hydroxy naphthoic acid, isethionic acid, lactobionic acid, mandelic acid, estolic acid, mucic acid, napsylic acid, muconic acid, p-nitromethanesulfonic acid, hexamic acid, pantothenic acid, monohydrogen phosphoric acid, dihydrogen phosphoric acid, salicylic acid, sulfamic acid, sulfanilic acid, methanesulfonic acid, or theoclic acid. In addition, the form of basic salt includes, for example, alkali and alkaline earth metal salts such as ammonium salts, lithium salts, sodium salts, potassium salts, magnesium salts and calcium salts; for example, salts with organic bases such as benzathine, N-methyl-D-glucamine, hydrabamine salts; and for example, salts with amino acids such as arginine and lysine. The salt form may also be converted to the free form by being treated with a suitable base or acid. The term "additional salt" includes solvates which the compound of Chemical Formula 1 and salts thereof can form. Such solvates are, for example, hydrates and alcoholates. The medicament or pharmaceutical composition according to an embodiment of the present invention may be for oral or parenteral administration, preferably for oral administration. In the case of parenteral administration, the medicament or pharmaceutical composition may be for parenteral administration such as intravenous injection, subcutaneous injection, intramuscular injection, intraperitoneal injection, endothelial administration, topical administration, intranasal administration, vaginal administration, intrapulmonary administration, or rectal administration. In the case of oral administration, the medicament or pharmaceutical composition of the present invention may be formulated in the form of powders, granules, tablets, pills, sugarcoated tablets, capsules, liquids, gels, syrups, suspensions, wafers, and the like according to the methods known in the art. The pharmaceutical composition according to an embodiment may be formulated to be uncoated or coated with the active agent or to be protected from degradation in the stomach. The composition may be administered by any device capable of transporting the active agent to a target cell. The route of administration may vary depending on the general condition and age of the subject to be treated, the nature of the treatment condition, and the active ingredient selected.

The suitable dosage of the medicament or pharmaceutical composition according to an embodiment of the present invention varies depending on factors such as formulation method, administration mode, and age, weight, sex, pathological condition, food, administration time, administration route, excretion rate and reaction sensitivity of the patient. An ordinarily skilled physician can readily determine and prescribe a dosage effective for the desired treatment or prophylaxis. The pharmaceutical composition according to an embodiment may be administered in one or several doses, for example, may be administered in divided doses from 1 to 4 times a day. The pharmaceutical composition according to an embodiment may contain 0.1 to 500 mg/kg (body weight), preferably 0.5 to 100 mg/kg (body weight) of the compound of Chemical Formula 1.

The medicament or pharmaceutical composition according to an embodiment of the present invention may be prepared in unit dose form by being formulated using a pharmaceutically acceptable carrier and/or filler or by being contained in a multiple-dose container according to a method that can be easily performed by a person of ordinary skill in the art to which the present invention pertains. In this case, the formulation may be in the form of a solution, suspension or emulsion in an oil or aqueous medium, may be in the form of an extract, powder, granule, tablet or capsule, and may additionally contain a dispersant or a stabilizing agent. The pharmaceutical composition may be administered in the form of a suppository, spray, ointment, cream, gel, inhalant or skin patch. The pharmaceutical composition may be prepared for administration to a mammal, more preferably for administration to a human.

The pharmaceutically acceptable carrier may be solid or liquid, and may be one or more selected from a filler, an antioxidant, a buffer, a bacteriostat, a dispersant, an adsorbent, a surfactant, a binder, a preservative, a disintegrant, a sweetening agent, a flavoring agent, a glidant, a release controlling agent, a wetting agent, a stabilizing agent, a suspending agent, or a lubricant. The pharmaceutically acceptable carrier may be selected from saline, sterile water, Ringer's solution, buffered saline, dextrose solution, maltodextrin solution, glycerol, ethanol, or any mixture thereof.

In an embodiment, as a suitable filler, sugars (for example, dextrose, sucrose, maltose and lactose), starch (for example, corn starch), sugar-alcohols (for example, mannitol, sorbitol, maltitol, erythritol and xylitol), starch hydrolysates (for example, dextrin and maltodextrin), cellulose or cellulose derivatives (for example, microcrystalline cellulose), or any mixture thereof may be used, but the filler is not limited thereto.

In an embodiment, as a suitable binder, magnesium aluminum silicate, povidone, copovidone, methylcellulose, hydroxymethylcellulose, hydroxypropylmethylcellulose, hydroxypropylcellulose, hydroxyethylcellulose, gelatin, gum, sucrose, starch paste, or any mixture thereof may be used, but the binder is not limited thereto.

In an embodiment, as a suitable preservative, benzoic acid, sodium benzoate, benzyl alcohol, butylated hydroxyanisole, butylated hydroxytoluene, chlorobutanol, gallate, hydroxybenzoate, EDTA, or any mixture thereof may be used, but the preservative is not limited thereto.

In an embodiment, as a suitable disintegrant, sodium starch glycolate, cross-linked polyvinyl pyrrolidone, cross-linked carboxymethylcellulose, starch, microcrystalline cellulose, or any mixture thereof may be used, but the disintegrant is not limited thereto.

In an embodiment, as a suitable sweetening agent, sucralose, saccharin, sodium or potassium or calcium saccharin, acesulfame potassium or sodium cyclamate, mannitol, fructose, sucrose, maltose, or any mixture thereof may be used, but the sweetening agent is not limited thereto.

In an embodiment, as a suitable glidant, silica, colloidal silicon dioxide, talc and the like may be used, but the glidant is not limited thereto.

In an embodiment, as a suitable lubricant, long chain fatty acids and their salts, for example, magnesium stearate and stearic acid, talc, glyceride wax, or any mixture thereof may be used, but the lubricant is not limited thereto.

The imidazopyrimidine or imidazotriazine compound of Chemical Formula 1 may be used together with an additional drug for alleviation or treatment of developmental disorder.

Accordingly, the medicament or pharmaceutical composition of the present invention may be a combination preparation containing the imidazopyrimidine or imidazotriazine compound of Chemical Formula 1 and such an additional drug as an active ingredient in a therapeutically effective amount.

Such additional drugs include, for example, stimulants (for example, methylphenidate, guanfacine, amphetamine, and lisdexamfetamine) and antidepressants (for example, fluoxetine, venlafaxine, sertraline, paroxetine, duloxetine, and escitalopram), and/or antipsychotics (for example, risperidone, quetiapine, aripiprazole, and olanzapine), antiepileptics (for example, cenobamate, carbamazepine, levetiracetam, lamotrigine, phenytoin, valproate, topiramate, phenobarbital, gabapentin, and vigabatrin), and anxiolytics (for example, chlordiazepoxide, diazepam, oxazepam, clonazepam, alprazolam, lorazepam, temazepam, flurazepam, triazolam, clorazepate, pregabalin, and buspirone).

In an embodiment, when the medicament or pharmaceutical composition of the present invention is the above-described combination preparation, the mixed weight ratio (a:b) of the imidazopyrimidine or imidazotriazine compound of Chemical Formula 1 [component (a)] to the additional drug [component (b)] in the combination preparation may be, for example, in a range of 1,000:1 to 1:1,000, or 500:1 to 1:500, or 100:1 to 1:100, or 50:1 to 1:50, or 10:1 to 1:10, but is not limited thereto.

Accordingly, the medicament or pharmaceutical composition according to an embodiment of the present invention may further contain one or more drugs selected from the group consisting of a stimulant, an antidepressant, an antipsychotic, an antiepileptic, and an anxiolytic.

More specifically, the stimulant may be one or more selected from the group consisting of methylphenidate, guanfacine, amphetamine, and lisdexamfetamine.

More specifically, the antidepressant may be one or more selected from the group consisting of fluoxetine, venlafaxine, sertraline, paroxetine, duloxetine, and escitalopram.

More specifically, the antipsychotic may be one or more selected from the group consisting of risperidone, quetiapine, aripiprazole, and olanzapine.

More specifically, the antiepileptic may be one or more selected from the group consisting of cenobamate, carbamazepine, levetiracetam, lamotrigine, phenytoin, valproate, topiramate, phenobarbital, gabapentin, and vigabatrin.

More specifically, the anxiolytic may be one or more selected from the group consisting of chlordiazepoxide, diazepam, oxazepam, clonazepam, alprazolam, lorazepam, temazepam, flurazepam, triazolam, clorazepate, pregabalin, and buspirone.

As used herein, the terms "prevent", "preventing", and "prevention" refer to reducing or eliminating the likelihood of contracting a disease.

As used herein, the terms "alleviate", "alleviating", and "alleviation" refer to relieving a disease and/or all or part of its attendant symptoms.

As used herein, the terms "treat", "treating", and "treatment" refer to eliminating a disease and/or all or part of its attendant symptoms.

As used herein, the term "subject" refers to animals, preferably mammals (for example, primates (for example, humans), cattle, sheep, goats, horses, dogs, cats, rabbits, rats, mice, and the like), most preferably humans, that are the objects of treatment, observation or experimentation.

As used herein, the term "therapeutically effective amount" refers to an amount of an active compound or pharmaceutical agent inducing a biological or medical response in a tissue system, animal or human, which is sought by a researcher, veterinarian, physician or other clinician and includes alleviating the symptoms of the disease or disorder to be treated.

As used herein, the term "composition" includes products containing specified ingredients in specified amounts and arbitrary products that result, directly or indirectly, from combinations of specified ingredients in specified amounts.

### [Advantageous Effects of Invention]

The medicament and pharmaceutical composition according to the present invention can efficiently prevent, alleviate or treat developmental disorder.

### [Brief Description of Drawings]

FIG. 1 is results showing the effect of long-term (6 weeks) administration of a test compound (2 mg/kg, twice a day) on reduced fear conditioning in a contextual fear conditioning experiment performed using an Fmrl gene knockout mouse model.
FIG. 2 is results showing the effect of long-term (6 weeks) administration of a test compound (2 mg/kg, twice a day) on an Fmrl gene knockout mouse model in an open field test.
FIG. 3 is results showing the effect of long-term (6 weeks) administration of a test compound (2 mg/kg, twice a day) on an Fmrl gene knockout mouse model in a marble burying test.

### [Description of Embodiments]

Hereinafter, the present invention will be described in more detail through Examples. However, these Examples are only for illustration of one or more embodiments, and the scope of the invention is not limited thereto.

### Preparation Example: Preparation of test compound

According to the method described in Example 113 of International Publication No. WO 2016/137260, 6-[4-fluoro-2-(trifluoromethyl)phenyl]-2-(2-pyridyloxymethyl)imidazo[1,2-a]pyrimidine (test compound) was prepared.

### Example 1: Anticonvulsant efficacy experiment on audiogenic seizure, contextual fear conditioning experiment, open field test, and marble burying test in Fmrl gene knockout mouse model

An Fmrl gene knockout mouse model was used as an animal model of fragile X syndrome. This model lacks FMRP protein due to disruption of the Fmrl gene. Typical phenotypes of this mouse model include audiogenic seizure, hyperlocomotor activity, cognitive deficit, concentration deficit disorder, and the like.

### Experimental animal

Male Fmrl C57BL/6J knock-out mice derived from the early group imported from Jackson Laboratories were used in the experiments. The experimental animals were raised in a light-controlled environment (illuminated for 12 hours/nonilluminated for 12 hours) and maintained at a temperature of 21°C ± 2°C and a relative humidity of about 55% ± 5%. Food and water were ad libitum fed. An anticonvulsant efficacy experiment on audiogenic seizure, a contextual fear conditioning experiment, an open field test, and a marble burying test in the Fmrl gene knockout mouse model was performed by Fragile X Syndrome Drug Validation Initiative (FRAXA-DVI). The mice were randomly divided into groups and the test was performed under illumination.

### Drug

The test compound was dissolved in 10% DMSO (dimethylsulfoxide) + 30% PEG 300 in DW (distilled water), and administered intraperitoneally at a dose of 2 mg/kg twice a day for 6 weeks. To a control group, vehicle (10% DMSO (dimethylsulfoxide) + 30% PEG 300 in DW (distilled water)) was administered.

### Test group

1. Wild-type mice - administered with vehicle
2. Fmrl gene knockout mice - administered with vehicle
3. Wild-type mice - administered with test compound at 2 mg/kg
4. Fmrl gene knockout mice - administered with test compound at 2 mg/kg
   Ten mice per group were used.
   Audiogenic seizure test
   After the vehicle or test compound was administered intraperitoneally at a dose of 2 mg/kg twice a day for 6 weeks, the mice were placed in a chamber and exposed to an alarm. The response of the mice during the test was evaluated numerically by the following criteria according to the intensity of seizure, and the survival rate was also observed.
   0: No response
   1: Wild running and jumping
   2: Clonic seizures
   3: Clonic-tonic seizures
   4: Tonic seizures
   5: Respiratory arrest

### Contextual fear conditioning experiment

After the vehicle or test compound was administered intraperitoneally at a dose of 2 mg/kg twice a day for 6 weeks, training and an efficacy test were performed, respectively. The chamber was cleaned with 70% ethanol every time mouse was changed. The mouse was placed in a conditioned chamber and an electric shock (unconditioned stimulus) was applied. To conduct a drug efficacy test on contextual memory, the mice were placed in the same chamber, and then left for 5 minutes without shock or other obstructions, and freezing behavior, defined as complete loss of movement except breathing, was observed.

### Open field test

After the vehicle or test compound was administered intraperitoneally at a dose of 2 mg/kg twice a day for 6 weeks, an open field test was performed to observe the efficacy behavior of mice who fearlessly stepped out to the center of the open field. Before the test, the mice were acclimatized to the laboratory and then placed in the center of the open field to measure their activity. The number of times the mouse crossed the center of the open field was measured by the breakage of the horizontal beam due to the mouse's movement. The test room was cleaned every time the mouse was changed.

### Marble burying test

After the vehicle or test compound was administered intraperitoneally at a dose of 2 mg/kg twice a day for 6 weeks, a hippocampal function-dependent marble burying test was performed. In a mouse cage, 16 transparent marbles were placed in a 4 × 4 arrangement so that 2/3 of the marble was submerged in the bedding. The mouse was placed and left for 30 minutes, then the number of marbles buried by the mouse was counted.

### Statistical analysis

After data analysis by ANOVA, the effect was defined as significant when p < 0.05. All results were expressed as mean ± SEM.

### Test result

The results of anticonvulsant efficacy test on audiogenic seizure using a test compound are summarized in Table 1.

**[Table 1] Results of anticonvulsant efficacy test on audiogenic seizure using test compound**

| Test group | Wild running | Seizures of 3 or more points | Survival rate | Vulnerability to audiogenic seizure |
|---|---|---|---|---|
| Wild type - vehicle | 0/10 | 0/10 | 10/10 | 0% |
| Fmrl gene knockout - vehicle | 9/10 | 8/10 | 2/10 | 90% |
| Wild type - test compound | 0/10 | 0/10 | 10/10 | 0% |
| Fmrl gene knockout - test compound | 6/10 | 5/10 | 5/10 | 60% |

In wild-type mice, no mice showed audiogenic seizure phenotype in the vehicle-administered group and the test compound-administered group. In the vehicle-administered group of Fmrl gene knockout mice, there were nine wild running mice, and eight mice showed seizures of 3 or more points among these, and the eight mice all died because of respiratory arrest. The vulnerability to audiogenic seizure is the proportion of wild running mice, and thus, the vulnerability to audiogenic seizure was 90% in the vehicle-treated group of Fmrl gene knockout mice. In the test compound-administered group of Fmrl gene knockout mice, there were six wild running mice, and five mice showed seizures of 3 or more points among these, and the five mice all died because of respiratory arrest. Hence, the vulnerability to audiogenic seizure was 60%. From this, it has been found that in Fmrl gene knockout mice, the vulnerability to audiogenic seizure of the test compound-administered group was decreased to 60% from 90%, which was the vulnerability to audiogenic seizure of the vehicle-administered group.

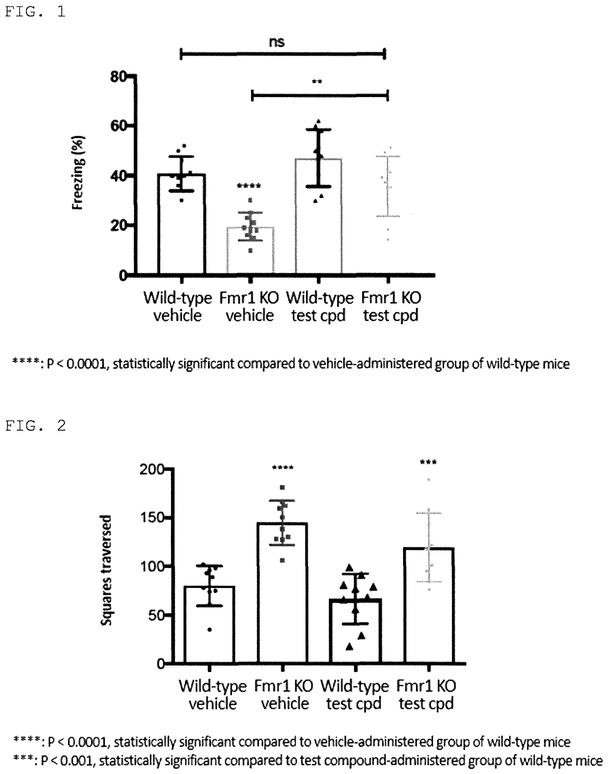

The percent of freezing behavior during the contextual fear conditioning experiment is illustrated in FIG. 1. As a result of ANOVA analysis, the vehicle-administered Fmrl gene knockout mice showed significantly decreased freezing behavior compared to the wild-type mice. In Fmrl gene knockout mice, the test compound-administered group showed significantly increased freezing behavior compared to the vehicle-administered group, and showed freezing behavior to a degree similar to that in the vehicle-administered wild-type mice. In other words, the result indicate that the fear learning ability in Fmrl gene knockout mice is improved.

As a result of the open field test, the number of times the mouse crossed the center is illustrated in FIG. 2. The vehicle-administered Fmrl gene knockout mice and the test compound-administered Fmrl gene knockout mice both had a P value of 0.0001 or less and showed a significant increase in movement compared to the wild-type mice.

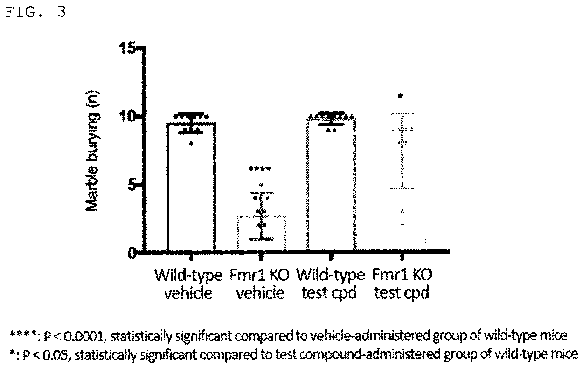

As a result of the marble burying test, the number of marbles buried is illustrated in FIG. 3. The vehicle-administered Fmrl gene knockout mice buried more marbles than the wild-type mice. The test compound-administered group of Fmrl gene knockout mice also showed a statistical difference compared to the wild-type mice, but the number of buried marbles in the test compound-administered group of Fmrl gene knockout mice increased compared to that in the vehicle-administered group of Fmrl gene knockout mice.

## Claims

1. A medicament for prevention, alleviation or treatment of developmental disorder, which comprises a therapeutically effective amount of an imidazopyrimidine or imidazotriazine compound of Chemical Formula 1, or a pharmaceutically acceptable salt, solvate or hydrate thereof: 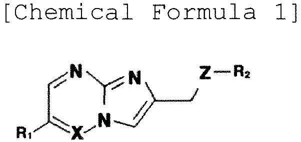 in Chemical Formula 1,
X is CH or N;
Z is O or S;
R₁ is C₆-C₁₂ aryl unsubstituted or substituted with one or more substituents selected from halo, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl, C₁-C₅ alkyl-C(O)O-C₁-C₅ alkyl, a 5- or 6-membered heterocycloalkyl-C₁-C₅ alkyl having 1 to 3 heteroatoms selected from N, O and S, and di (C₁-C₅ alkyl)amino-C₁-C₅ alkyl; or 5- to 12-membered unsaturated heterocyclyl having 1 to 5 heteroatoms selected from N, 0 and S, unsubstituted or substituted with one or more substituents selected from halo, hydroxy, C₁-C₅ alkyl, C₁-C₅ alkoxy, and halo-C₁-C₅ alkyl;
R₂ is C₆-C₁₂ aryl unsubstituted or substituted with one or more substituents selected from halo, deuterium, hydroxy and C₁-C₅ alkyl; or 5- to 12-membered unsaturated heterocyclyl having 1 to 3 heteroatoms selected from N, O and S, unsubstituted or substituted with one or more substituents selected from halo and C₁-C₅ alkyl.

2. The medicament according to claim 1, wherein R₁ is phenyl unsubstituted or substituted with 1 to 3 substituents selected from halo, hydroxy, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl and C₁-C₅ alkyl-C(O)O-C₁-C₅ alkyl; or 5- to 10-membered unsaturated heterocyclyl having 1 to 3 heteroatoms selected from N, O and S, unsubstituted or substituted with 1 to 3 substituents selected from halo, hydroxy, C₁-C₅ alkyl, C₁-C₅ alkoxy and halo-C₁-C₅ alkyl.

3. The medicament according to claim 1, wherein R₂ is phenyl unsubstituted or substituted with 1 to 5 substituents selected from halo, deuterium, hydroxy and C₁-C₅ alkyl; or 5- or 6-membered heteroaryl having 1 to 3 heteroatoms selected from N, O and S, unsubstituted or substituted with 1 to 3 substituents selected from halo and C₁-C₅ alkyl.

4. The medicament according to claim 1, wherein
X is CH or N;
Z is 0;
R₁ is phenyl unsubstituted or substituted with 1 to 3 substituents selected from halo, hydroxy, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl and C₁-C₅ alkyl-C(O)O-C₁-C₅ alkyl; or 5- to 9-membered unsaturated heterocyclyl having 1 or 2 heteroatoms selected from N, O and S, unsubstituted or substituted with 1 or 2 substituents selected from halo, hydroxy, C₁-C₅ alkyl, C₁-C₅ alkoxy and halo-C₁-C₅ alkyl; and
R₂ is phenyl unsubstituted or substituted with 1 to 5 substituents selected from halo, deuterium, hydroxy and C₁-C₅ alkyl; or 6-membered heteroaryl having 1 or 2 nitrogen atoms, unsubstituted or substituted with 1 or 2 substituents selected from halo and C₁-C₅ alkyl.

5. The medicament according to claim 1, wherein
R₁ is phenyl unsubstituted or substituted with 1 to 3 substituents selected from halo, hydroxy, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl and C₁-C₅ alkyl-C(O)O-C₁-C₅ alkyl; 1,3-benzodioxolyl unsubstituted or substituted with 1 or 2 halo; or pyridyl or pyrimidinyl unsubstituted or substituted with 1 or 2 substituents selected from halo, hydroxy, C₁-C₅ alkyl, C₁-C₅ alkoxy and halo-C₁-C₅ alkyl, and
R₂ is phenyl unsubstituted or substituted with 1 to 5 substituents selected from halo, deuterium, hydroxy and C₁-C₅ alkyl; or pyridyl unsubstituted or substituted with 1 or 2 substituents selected from halo and C₁-C₅ alkyl.

6. The medicament according to claim 1, wherein
X is CH;
Z is O;
R₁ is phenyl unsubstituted or substituted with 1 to 3 substituents selected from halo, hydroxy, C₁-C₅ alkyl, C₁-C₅ alkoxy, C₁-C₅ alkylthio, amino, halo-C₁-C₅ alkyl, hydroxy-C₁-C₅ alkyl and C₁-C₅ alkoxy-C₁-C₅ alkyl; and
R₂ is pyridyl unsubstituted or substituted with 1 or 2 substituents selected from halo and C₁-C₅ alkyl.

7. The medicament according to claim 1, wherein the imidazopyrimidine or imidazotriazine compound of Chemical Formula 1 is selected from the following compounds:
6-(4-fluorophenyl)-2-(pyridin-2-yloxymethyl)imidazo[1,2-a]pyrimidine;
6-[4-fluoro-2-(trifluoromethyl)phenyl]-2-(2-pyridyloxymethyl)imidazo[1,2-a]pyrimidine;
6-(4-fluoro-2-methyl-phenyl)-2-[(5-fluoro-2-pyridyl)oxymethyl)imidazo[1,2-a]pyrimidine;
6-(4-fluorophenyl)-2-[(5-fluoro-2-pyridyl)oxymethyl)imidazo[1,2-a]pyrimidine;
[5-fluoro-2-[2-(2-pyridyloxymethyl)imidazo[1,2-a]pyrimidin-6-yl]phenyl]methanol; and
6-[4-fluoro-2-(fluoromethyl)phenyl]-2-(2-pyridyloxymethyl)imidazo[1,2-a]pyrimidine.

8. The medicament according to claim 1, wherein the developmental disorder is selected from the group consisting of pervasive developmental disorder, autism, autistic spectrum disorder, fragile X syndrome, a developmental disorder caused by fragile X syndrome, a developmental disorder exhibiting symptoms similar to those of fragile X syndrome, Angelman syndrome, a developmental disorder caused by Angelman syndrome, a developmental disorder exhibiting symptoms similar to those of Angelman syndrome, Rett syndrome, a developmental disorder caused by Rett syndrome, a developmental disorder exhibiting symptoms similar to those of Rett syndrome, fragile X-associated tremor/ataxia syndrome (FXTAS), Asperger's syndrome, a developmental disorder caused by Asperger's syndrome, a developmental disorder exhibiting symptoms similar to those of Asperger's syndrome, childhood disintegrative disorder, a developmental disorder caused by childhood disintegrative disorder, a developmental disorder exhibiting symptoms similar to those of childhood disintegrative disorder, Landau-Kleffner syndrome, a developmental disorder caused by Landau-Kleffner syndrome, a developmental disorder exhibiting symptoms similar to those of Landau-Kleffner syndrome, Prader-Willi syndrome, a developmental disorder caused by Prader-Willi syndrome, a developmental disorder exhibiting symptoms similar to those of Prader-Willi syndrome, 22q11.2 deletion syndrome, a developmental disorder caused by 22q11.2 deletion syndrome, a developmental disorder exhibiting symptoms similar to those of 22q11.2 deletion syndrome, tardive dyskinesia, a developmental disorder caused by tardive dyskinesia, a developmental disorder exhibiting symptoms similar to those of tardive dyskinesia, seizure disorder, a developmental disorder caused by seizure disorder, a developmental disorder exhibiting symptoms similar to those of seizure disorder, Williams syndrome, a developmental disorder caused by Williams syndrome, and a developmental disorder exhibiting symptoms similar to those of Williams syndrome.

9. The medicament according to claim 1, wherein the developmental disorder is selected from the group consisting of fragile X syndrome, a developmental disorder caused by fragile X syndrome, a developmental disorder exhibiting symptoms similar to those of fragile X syndrome, Angelman syndrome, a developmental disorder caused by Angelman syndrome, a developmental disorder exhibiting symptoms similar to those of Angelman syndrome, Rett syndrome, a developmental disorder caused by Rett syndrome, and a developmental disorder exhibiting symptoms similar to those of Rett syndrome.

10. The medicament according to claim 1, wherein the developmental disorder is selected from the group consisting of fragile X syndrome, a developmental disorder caused by fragile X syndrome, and a developmental disorder exhibiting symptoms similar to those of fragile X syndrome.

11. The medicament according to claim 1, which is used for preventing, alleviating or treating symptoms of developmental disorder.

12. The medicament according to claim 11, wherein the symptoms of developmental disorder are developmental delays, learning disability, intellectual disability, socio-behavioral disorder or seizures.

13. The medicament according to claim 1, which is prepared for administration to a mammal.

14. The medicament according to claim 1, comprising 0.1 to 500 mg/kg (body weight) of the imidazopyrimidine or imidazotriazine compound of Chemical Formula 1.

15. The medicament according to claim 1, which is for oral administration or for parenteral administration selected from the group consisting of intravenous injection, subcutaneous injection, intramuscular injection, intraperitoneal injection, endothelial administration, topical administration, intranasal administration, vaginal administration, intrapulmonary administration and rectal administration.

16. The medicament according to claim 1, further comprising one or more drugs selected from the group consisting of a stimulant, an antidepressant, an antipsychotic, an antiepileptic, and an anxiolytic.

17. A pharmaceutical composition for prevention, alleviation or treatment of developmental disorder, which comprises a therapeutically effective amount of an imidazopyrimidine or imidazotriazine compound of Chemical Formula 1, or a pharmaceutically acceptable salt, solvate or hydrate thereof, and additionally one or more pharmaceutically acceptable carriers: 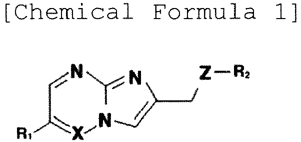 in Chemical Formula 1,
X is CH or N;
Z is 0 or S;
R₁ is C₆-C₁₂ aryl unsubstituted or substituted with one or more substituents selected from halo, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-Ci-Cs alkyl, carbamoyloxy-C₁-C₅ alkyl, C₁-C₅ alkyl-C(O)O-C₁-C₅ alkyl, a 5- or 6-membered heterocycloalkyl-C₁-C₅ alkyl having 1 to 3 heteroatoms selected from N, 0 and S, and di(C₁-C₅ alkyl) amino-Ci-Cs alkyl; or 5- to 12-membered unsaturated heterocyclyl having 1 to 5 heteroatoms selected from N, 0 and S, unsubstituted or substituted with one or more substituents selected from halo, hydroxy, C₁-C₅ alkyl, C₁-C₅ alkoxy, and halo-C₁-C₅ alkyl;
R₂ is C₆-C₁₂ aryl unsubstituted or substituted with one or more substituents selected from halo, deuterium, hydroxy and C₁-C₅ alkyl; or 5- to 12-membered unsaturated heterocyclyl having 1 to 3 heteroatoms selected from N, O and S, unsubstituted or substituted with one or more substituents selected from halo and C₁-C₅ alkyl.

18. The pharmaceutical composition according to claim 17, wherein the developmental disorder is selected from the group consisting of pervasive developmental disorder, autism, autistic spectrum disorder, fragile X syndrome, a developmental disorder caused by fragile X syndrome, a developmental disorder exhibiting symptoms similar to those of fragile X syndrome, Angelman syndrome, a developmental disorder caused by Angelman syndrome, a developmental disorder exhibiting symptoms similar to those of Angelman syndrome, Rett syndrome, a developmental disorder caused by Rett syndrome, a developmental disorder exhibiting symptoms similar to those of Rett syndrome, fragile X-associated tremor/ataxia syndrome (FXTAS), Asperger's syndrome, a developmental disorder caused by Asperger's syndrome, a developmental disorder exhibiting symptoms similar to those of Asperger's syndrome, childhood disintegrative disorder, a developmental disorder caused by childhood disintegrative disorder, a developmental disorder exhibiting symptoms similar to those of childhood disintegrative disorder, Landau-Kleffner syndrome, a developmental disorder caused by Landau-Kleffner syndrome, a developmental disorder exhibiting symptoms similar to those of Landau-Kleffner syndrome, Prader-Willi syndrome, a developmental disorder caused by Prader-Willi syndrome, a developmental disorder exhibiting symptoms similar to those of Prader-Willi syndrome, 22q11.2 deletion syndrome, a developmental disorder caused by 22q11.2 deletion syndrome, a developmental disorder exhibiting symptoms similar to those of 22q11.2 deletion syndrome, tardive dyskinesia, a developmental disorder caused by tardive dyskinesia, a developmental disorder exhibiting symptoms similar to those of tardive dyskinesia, seizure disorder, a developmental disorder caused by seizure disorder, a developmental disorder exhibiting symptoms similar to those of seizure disorder, Williams syndrome, a developmental disorder caused by Williams syndrome, and a developmental disorder exhibiting symptoms similar to those of Williams syndrome.

19. The pharmaceutical composition according to claim 17, wherein the developmental disorder is selected from the group consisting of fragile X syndrome, a developmental disorder caused by fragile X syndrome, a developmental disorder exhibiting symptoms similar to those of fragile X syndrome, Angelman syndrome, a developmental disorder caused by Angelman syndrome, a developmental disorder exhibiting symptoms similar to those of Angelman syndrome, Rett syndrome, a developmental disorder caused by Rett syndrome, and a developmental disorder exhibiting symptoms similar to those of Rett syndrome.

20. The pharmaceutical composition according to claim 17, wherein the developmental disorder is selected from the group consisting of fragile X syndrome, a developmental disorder caused by fragile X syndrome, and a developmental disorder exhibiting symptoms similar to those of fragile X syndrome.

21. The pharmaceutical composition according to claim 17, which is used for preventing, alleviating or treating symptoms of developmental disorder.

22. The pharmaceutical composition according to claim 21, wherein the symptoms of developmental disorder are developmental delays, learning disability, intellectual disability, socio-behavioral disorder or seizures.

23. The pharmaceutical composition according to claim 17, which is prepared for administration to a mammal.

24. The pharmaceutical composition according to claim 17, comprising 0.1 to 500 mg/kg (body weight) of the imidazopyrimidine or imidazotriazine compound of Chemical Formula 1.

25. The pharmaceutical composition according to claim 17, which is for oral administration or for parenteral administration selected from the group consisting of intravenous injection, subcutaneous injection, intramuscular injection, intraperitoneal injection, endothelial administration, topical administration, intranasal administration, vaginal administration, intrapulmonary administration and rectal administration.

26. The pharmaceutical composition according to claim 17, wherein the pharmaceutically acceptable carrier is one or more selected from a filler, an antioxidant, a buffer, a bacteriostat, a dispersant, an adsorbent, a surfactant, a binder, a preservative, a disintegrant, a sweetening agent, a flavoring agent, a glidant, a release controlling agent, a wetting agent, a stabilizing agent, a suspending agent and a lubricant.

27. The pharmaceutical composition according to claim 17, further comprising one or more drugs selected from the group consisting of a stimulant, an antidepressant, an antipsychotic, an antiepileptic, and an anxiolytic.

28. A method for prevention, alleviation or treatment of developmental disorder in a subject, which comprises administering to the subject a therapeutically effective amount of an imidazopyrimidine or imidazotriazine compound of Chemical Formula 1, or a pharmaceutically acceptable salt, solvate or hydrate thereof: 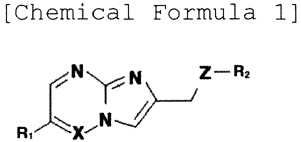 in Chemical Formula 1,
X is CH or N;
Z is O or S;
R₁ is C₆-C₁₂ aryl unsubstituted or substituted with one or more substituents selected from halo, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl, C₁-C₅ alkyl-C(O)O-C₁-C₅ alkyl, a 5- or 6-membered heterocycloalkyl-Ci-C₅ alkyl having 1 to 3 heteroatoms selected from N, O and S, and di(C₁-C₅ alkyl) amino-C₁-C₅ alkyl; or 5- to 12-membered unsaturated heterocyclyl having 1 to 5 heteroatoms selected from N, O and S, unsubstituted or substituted with one or more substituents selected from halo, hydroxy, C₁-C₅ alkyl, C₁-C₅ alkoxy, and halo-Ci-Cs alkyl;
R₂ is C₆-C₁₂ aryl unsubstituted or substituted with one or more substituents selected from halo, deuterium, hydroxy and C₁-C₅ alkyl; or 5- to 12-membered unsaturated heterocyclyl having 1 to 3 heteroatoms selected from N, O and S, unsubstituted or substituted with one or more substituents selected from halo and C₁-C₅ alkyl.

29. The method according to claim 28, wherein the developmental disorder is selected from the group consisting of pervasive developmental disorder, autism, autistic spectrum disorder, fragile X syndrome, a developmental disorder caused by fragile X syndrome, a developmental disorder exhibiting symptoms similar to those of fragile X syndrome, Angelman syndrome, a developmental disorder caused by Angelman syndrome, a developmental disorder exhibiting symptoms similar to those of Angelman syndrome, Rett syndrome, a developmental disorder caused by Rett syndrome, a developmental disorder exhibiting symptoms similar to those of Rett syndrome, fragile X-associated tremor/ataxia syndrome (FXTAS), Asperger's syndrome, a developmental disorder caused by Asperger's syndrome, a developmental disorder exhibiting symptoms similar to those of Asperger's syndrome, childhood disintegrative disorder, a developmental disorder caused by childhood disintegrative disorder, a developmental disorder exhibiting symptoms similar to those of childhood disintegrative disorder, Landau-Kleffner syndrome, a developmental disorder caused by Landau-Kleffner syndrome, a developmental disorder exhibiting symptoms similar to those of Landau-Kleffner syndrome, Prader-Willi syndrome, a developmental disorder caused by Prader-Willi syndrome, a developmental disorder exhibiting symptoms similar to those of Prader-Willi syndrome, 22q11.2 deletion syndrome, a developmental disorder caused by 22q11.2 deletion syndrome, a developmental disorder exhibiting symptoms similar to those of 22q11.2 deletion syndrome, tardive dyskinesia, a developmental disorder caused by tardive dyskinesia, a developmental disorder exhibiting symptoms similar to those of tardive dyskinesia, seizure disorder, a developmental disorder caused by seizure disorder, a developmental disorder exhibiting symptoms similar to those of seizure disorder, Williams syndrome, a developmental disorder caused by Williams syndrome, and a developmental disorder exhibiting symptoms similar to those of Williams syndrome.

30. The method according to claim 28, wherein the developmental disorder is selected from the group consisting of fragile X syndrome, a developmental disorder caused by fragile X syndrome, a developmental disorder exhibiting symptoms similar to those of fragile X syndrome, Angelman syndrome, a developmental disorder caused by Angelman syndrome, a developmental disorder exhibiting symptoms similar to those of Angelman syndrome, Rett syndrome, a developmental disorder caused by Rett syndrome, and a developmental disorder exhibiting symptoms similar to those of Rett syndrome.

31. The method according to claim 28, wherein the developmental disorder is selected from the group consisting of fragile X syndrome, a developmental disorder caused by fragile X syndrome, and a developmental disorder exhibiting symptoms similar to those of fragile X syndrome.

32. The method according to claim 28, which is for preventing, alleviating or treating symptoms of developmental disorder.

33. The method according to claim 32, wherein the symptoms of developmental disorder are developmental delays, learning disability, intellectual disability, socio-behavioral disorder or seizures.

34. The method according to claim 28, wherein the subject is a mammal.

35. The method according to claim 28, wherein 0.1 to 500 mg/kg (body weight) of the imidazopyrimidine or imidazotriazine compound of Chemical Formula 1 is administered.

36. The method according to claim 28, wherein one or more drugs selected from the group consisting of a stimulant, an antidepressant, an antipsychotic, an antiepileptic, and an anxiolytic are additionally administered.

37. Use of an imidazopyrimidine or imidazotriazine compound of Chemical Formula 1, or a pharmaceutically acceptable salt, solvate or hydrate thereof for prevention, alleviation or treatment of developmental disorder: 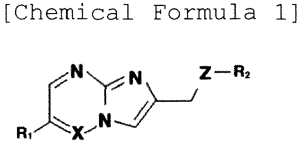 in Chemical Formula 1,
X is CH or N;
Z is 0 or S;
R₁ is C₆-C₁₂ aryl unsubstituted or substituted with one or more substituents selected from halo, C₁-C₅ alkyl, C₁-C₅ alkoxy, C₁-C₅ alkylthio, amino, di(C₁-C₅ alkyl)amino, cyano, formyl, halo-C₁-C₅ alkyl, hydroxy-C₁-C₅ alkyl, C₁-C₅ alkoxy-C₁-C₅ alkyl, carbamoyloxy-C₁-C₅ alkyl, C₁-C₅ alkyl-C(O)O-C₁-C₅ alkyl, a 5- or 6-membered heterocycloalkyl-C₁-C₅ alkyl having 1 to 3 heteroatoms selected from N, O and S, and di (C₁-C₅ alkyl) amino-C₁-C₅ alkyl; or 5- to 12-membered unsaturated heterocyclyl having 1 to 5 heteroatoms selected from N, O and S, unsubstituted or substituted with one or more substituents selected from halo, hydroxy, C₁-C₅ alkyl, C₁-C₅ alkoxy, and halo-C₁-C₅ alkyl;
R₂ is C₆-C₁₂ aryl unsubstituted or substituted with one or more substituents selected from halo, deuterium, hydroxy and C₁-C₅ alkyl; or 5- to 12-membered unsaturated heterocyclyl having 1 to 3 heteroatoms selected from N, O and S, unsubstituted or substituted with one or more substituents selected from halo and C₁-C₅ alkyl.

38. The use according to claim 37, wherein the developmental disorder is selected from the group consisting of pervasive developmental disorder, autism, autistic spectrum disorder, fragile X syndrome, a developmental disorder caused by fragile X syndrome, a developmental disorder exhibiting symptoms similar to those of fragile X syndrome, Angelman syndrome, a developmental disorder caused by Angelman syndrome, a developmental disorder exhibiting symptoms similar to those of Angelman syndrome, Rett syndrome, a developmental disorder caused by Rett syndrome, a developmental disorder exhibiting symptoms similar to those of Rett syndrome, fragile X-associated tremor/ataxia syndrome (FXTAS), Asperger's syndrome, a developmental disorder caused by Asperger's syndrome, a developmental disorder exhibiting symptoms similar to those of Asperger's syndrome, childhood disintegrative disorder, a developmental disorder caused by childhood disintegrative disorder, a developmental disorder exhibiting symptoms similar to those of childhood disintegrative disorder, Landau-Kleffner syndrome, a developmental disorder caused by Landau-Kleffner syndrome, a developmental disorder exhibiting symptoms similar to those of Landau-Kleffner syndrome, Prader-Willi syndrome, a developmental disorder caused by Prader-Willi syndrome, a developmental disorder exhibiting symptoms similar to those of Prader-Willi syndrome, 22q11.2 deletion syndrome, a developmental disorder caused by 22q11.2 deletion syndrome, a developmental disorder exhibiting symptoms similar to those of 22q11.2 deletion syndrome, tardive dyskinesia, a developmental disorder caused by tardive dyskinesia, a developmental disorder exhibiting symptoms similar to those of tardive dyskinesia, seizure disorder, a developmental disorder caused by seizure disorder, a developmental disorder exhibiting symptoms similar to those of seizure disorder, Williams syndrome, a developmental disorder caused by Williams syndrome, and a developmental disorder exhibiting symptoms similar to those of Williams syndrome.

39. The use according to claim 37, wherein the developmental disorder is selected from the group consisting of fragile X syndrome, a developmental disorder caused by fragile X syndrome, a developmental disorder exhibiting symptoms similar to those of fragile X syndrome, Angelman syndrome, a developmental disorder caused by Angelman syndrome, a developmental disorder exhibiting symptoms similar to those of Angelman syndrome, Rett syndrome, a developmental disorder caused by Rett syndrome, and a developmental disorder exhibiting symptoms similar to those of Rett syndrome.

40. The use according to claim 37, wherein the developmental disorder is selected from the group consisting of fragile X syndrome, a developmental disorder caused by fragile X syndrome, and a developmental disorder exhibiting symptoms similar to those of fragile X syndrome.

41. The use according to claim 37, which is for preventing, alleviating or treating symptoms of developmental disorder.

42. The use according to claim 41, wherein the symptoms of developmental disorder are developmental delays, learning disability, intellectual disability, socio-behavioral disorder or seizures.

43. The use according to claim 37, which is for administration to a mammal.

44. The use according to claim 37, wherein 0.1 to 500 mg/kg (body weight) of the imidazopyrimidine or imidazotriazine compound of Chemical Formula 1 is used.

45. The use according to claim 37, wherein one or more drugs selected from the group consisting of a stimulant, an antidepressant, an antipsychotic, an antiepileptic, and an anxiolytic are additionally used.
